# EUROPEAN PATENT APPLICATION

(11) **EP 3 210 527 A1**
(43) Date of publication of application: **30.08.2017**
(21) Application number: 16157784.6
(22) Date of filing: 29.02.2016
(51) Int. Cl.: A61B 5/00, A61B 5/04, G06F 19/00, A61N 1/362

(54) **REMOTE PATIENT DATA MONITORING**

(71) Applicant: Covidien AG, 8212 Neuhausen am Rheinfall (CH)
(72) Inventor: van den Broek, Waldy, 6074 DP Melick (NL); Hoeff, Eelco, 1261 MC Blaricum (NL); Pans, Ivo, 3705 AN Zeist (NL); Verspui, Laura, 2021 DA Haarlem (NL); Montechiaro, Federico, 6854 Ligornetto, Ticino (CH); García de Pablo, Maria M., 28006 Madrid (ES); González, Rafael Moreno, 28863 Cobena, Madrid (ES)
(74) Representative: Olsson, Carl H.S.

(57) **Abstract**

Method and system for analyzing patient data transmitted from a patient device (1) to a healthcare center/professional (3) via a secured host server and communication network, wherein the patient data are classified (2) into risk level categories based on their clinical relevance, defined by certain criteria set by the healthcare center/professional (3).

## Description

### Field of the invention

The invention relates to a method and a system for monitoring healthcare services.

### Background of the invention

A few specialized medical device companies manufacture implantable cardiac devices for the diagnosis and therapy of cardiac rhythm diseases (hereafter referred to as 'implant' or 'implants') that are capable of automatically sending data to hospitals.

Health care professionals in these hospitals, with certain permissions, may access this data on a secured website without having to see the patient face-to-face, unless they consider this to be necessary. The implants are connected to the secured website through monitoring devices sending the data to a database which is hosted by the medical device company acting as the host server. The database in the host server is accessible by these healthcare professionals through the secured website.

Medtronic is an example of a medical device company manufacturing implants such as implantable therapeutic cardiac devices e.g., pacemakers (IPGs), implantable cardioverter defibrillators (ICDs) and implantable cardiac resynchronization therapy devices (CRTs) as well as implantable diagnostic devices, e.g., LINQ, an implantable cardiac monitoring system constantly recording the ECG and the heart beats.

These implants, whether therapeutic or diagnostic, transmit patient data to a monitoring device which is connected to a database (Carelink) hosted by Medtronic acting as a data processor. The database is accessible by the treating physicians and hospitals through the secured website, i.e., Carelink. Medtronic enables the remote connectivity between cardiac patients and treating physicians and hospitals, and allows for data to transfer from the patient to the physician and be stored on the Medtronic server.

The vast majority of these data transmissions from the patient's implant to the hospital do not contain any information that would require any clinical actions by the treating physician. Studies related to the present invention have shown that up to 80 % of the transmitted data only confirm that the implant is working well and around 20 % contain information that would trigger the physician to take any clinical actions. Nevertheless, the physician has to go through all the data transmitted to be able to identify those data (20 %) where clinical actions have to be taken. Consequently, the physician is spending a large amount of time assessing 'useless' information (up to 80%) with no or little clinical value.

An object of the present invention is to reduce the time physicians and healthcare professionals are spending on assessing these transmitted data and consequently 'free' up their time allowing them to use the time for patients in need of attention (e.g., face-to-face visits, surgical operations etc.)

### Summary of invention

This object is achieved by a data analyzing service classifying transmitted patient data based on their clinical relevance using selected criteria (based on agreed protocol with treating physicians) for assessing their clinical relevance, whereupon the analyzed data are provided through Carelink to the treating healthcare professionals (e.g., physicians, cardiologists, neurologists) in the hospital.

These triaging services include highlighting those patients, based on agreed protocols with the treating physicians, that show clinically relevant cardiac data to allow the treating physician to prioritize their clinical actions and allow them to act quickly on urgent cases, while reducing the time spent on those patients showing non-relevant clinical or false-positive data, i.e. data showing that the implant is working well. The analyzing/triaging services also include prompt communication to healthcare professionals in the hospital about these urgent cases through emails and/or phone calls, so that they can react to them according to the treating physician's judgments.

The triaging services according to the present invention have several advantages such as less time spent by healthcare professionals reviewing CareLink data, allowing them to use the time for patients in need of attention (e.g. for implants, ablations, or face to face visits). This would be achieved since around 80% of the transmitted data is not clinically relevant, and without the analyzing services according to the invention, the hospitals would still have to screen all of the data. Thanks to these analyzing services according to the invention, the healthcare professionals can focus on the 20% of the patient data that would require a prompt clinical judgment and potential intervention.

A further advantage of the analyzing/triaging services according to the invention is a significantly shorter reaction time to clinically relevant data allowing the deliverance of better quality of care for those patients who would need urgent attention.

### Brief description of the drawings

Figure 1 is an illustration of the analyzing/triaging services according to the present invention;
Figure 2 discloses certain classifications of transmissions reviewed by a monitoring center using the triaging services according to the invention.

### Description of the invention

In the following description a healthcare center should be defined broadly as any hospital, healthcare institution or health clinic etc. and healthcare professionals should be defined broadly as for example nurses, physicians, cardiologists, neurologists, medical doctors and other people entitled to make clinical assessments and medical decisions related to patients. It should also be realized that the implant according to the invention would not necessarily need to be a cardiac device but could be any type of communicative implantable device in a human or animal body, e.g. implantable hearing or visual aid, brain or spine implant etc.

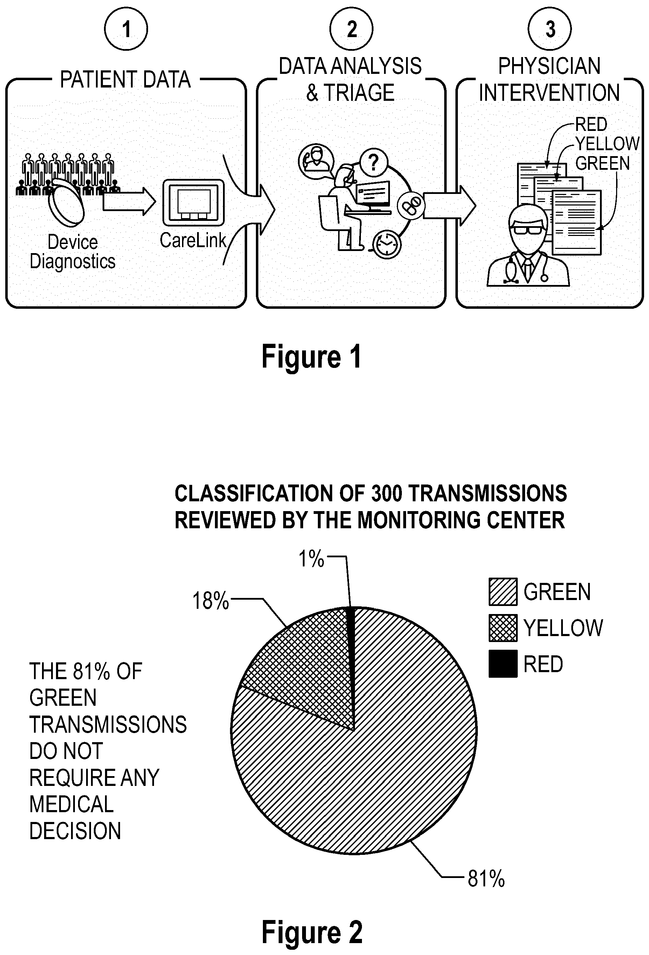

Figure 1 discloses the communication system between the patient's implant (1) and the healthcare professional (3) in a hospital. The implant (Device Diagnostics) sends patient data through monitoring devices (not shown) to a database which is hosted by the medical device company (Medtronic). The database in the host server is accessible by the healthcare professionals (3) through the secured website (Carelink). One embodiment of the invention resides in the intermediate analysis/triaging step (2) in figure 1 in which these patient data are analyzed by certified technicians, MDs or nurses, hereafter referred to as 'technicians'. The technicians (2) access the patient data in the database and classify the data based on their clinical relevance using selected criteria which are based on agreed protocol with the treating hospital/healthcare professionals (3). These analyzed/triaged and classified patient data are then uploaded by the technicians in the database, whereupon the triaged data will be accessible through the secured website (Carelink) to the treating physicians in the hospital (3).

In a preferred embodiment of the invention, the patient data are classified by the triaging service (technicians - 2) in three categories based on the clinical relevance of the data. The first category represents patient data with low risk, the second category represents patient data which is non-acute and the third category represents patient data which is acute. It should be emphasized that the classification of the patient data in these categories is carried out by the triaging technicians using strict criteria which are agreed upon by the treating hospital (3).

The first category with low risk carries data that the implants are working as intended, i.e., all clinical patient data, e.g., heartbeats, amplitudes and configuration in time and space of ECG's QRS -complex etc. are within the normal interval based on the patient's age and other patient specifics. Studies related to the invention have shown that this category would represent around 80 % of transmitted data (figure 2-green). This low risk category could preferably be communicated to the healthcare professional with a green color coding, and only once a week in a single email, to notify physician about its low risk.

The non-acute category contains data which are clinically relevant, e.g., heartbeats or amplitudes and configuration in time and space of ECG's QRS-complex, duration in time of heart arrhythmia etc. are slightly deviating from the normal interval based on the specifics of the patient. This non-acute risk category should preferably be communicated to the healthcare professional with a yellow color coding, and the following day in an email, to notify the healthcare professional about the higher risk. Studies according to the invention have shown that this category represents less than 20 % of the transmitted data (figure 2-yellow).

The acute category contains data which are clinically very relevant, e.g., heartbeats or amplitudes and configuration in time and space of ECG's QRS-complex are clearly outside the normal intervals (duration in time of heart arrhythmia, tachycardia faster than programmed levels and a longer duration than programmed levels, brady arrhythmia, brady cardia slower than programmed levels, AV block etc.) based on the specifics and the age of the patient. This acute risk category should preferably be communicated to the physician with a red color coding to notify the physician about the acute risk. For this acute risk category, an email should be sent to the hospital and a phone call should be made during the same day. Studies related to the present invention have shown that this category represent around 1 % of transmitted patient data (figure 2-red).

In a preferred embodiment of the present invention, these categories ('low risk', 'non-acute', 'acute') are communicated in prioritized emails by a traffic light coding, e.g., green, yellow and red. The green color coded email representing the low risk situation would be sent out once a week clustered in a single email, the yellow color coded email representing the non-acute situation would be sent out the next working day, and the red color coded email representing the acute situation would be sent out during the same working day.

In another embodiment of the present invention, the triaging/analyzing step (2) is carried out by an analyzing machine/device, such as a computer, automatically classifying the patient data in low risk, non-acute and acute categories based on the given criteria. The computer will then assess the ECG diagram and other patient data and based on the set criteria, will automatically calculate and classify the data into the required risk level categories. In addition the computer will take different actions based on category levels, and if for example the level is acute, the computer will send out a 'red color coded' email and make a phone call to the treating healthcare professional /physician within the required time limit.

In yet another embodiment the computer carries out the analyzing step as set out above and classifies the data into the required categories which is thereafter checked by the analyzing technicians (2). In this embodiment the computer is only a support or a back-up tool for the analyzing technicians enhancing the quality of the overall assessment.

The above embodiments should be regarded as illustrative rather than restrictive, and it should be appreciated that variations may be made in those embodiments by a person skilled in the art without departing from the scope of the present invention as defined in the following claims.

## Claims

1. Method for analyzing patient data transmitted from a patient device (1) to a healthcare center/professional (3) via a secured host server and communication network, **characterized in that**, said data are classified (2) into risk level categories based on the clinical relevance of said data, wherein said clinical relevance is defined by certain criteria, and **in that** said healthcare center/professional (3), based on said risk level category of said data, makes a prioritized decision on the further medical treatment of said patient.

2. Method according to claim 1, **characterized in that**, an analyzing/triaging service (2) analyzes the data in said host server and classifies it into said risk level categories based on said criteria, the criteria of which are set by said healthcare center/professional (3) and/or said analyzing/triaging service (2).

3. Method according to claim 2, **characterized in that**, said patient device is an implant (1) and **in that** said analyzing/triaging service (2) accesses said patient data from said host server, analyzes said data, classifies said data and uploads said data to said host server, whereby said classified data on said host server are accessible to said healthcare center/professional (3) via a secured website.

4. Method according to any of claims 2 or 3, **characterized in that**, said analyzing/triaging service (2), based on said risk level category of said data (low, non-acute, acute), contacts said healthcare center/professional via email and/or phone, wherein a higher risk level (acute, non-acute) implies a more urgent attention to the healthcare center/professional (3).

5. Method according to any of claims 2 to 4, **characterized in that**, said analyzing/triaging service (2) consists of a team of technicians specialized in carrying out the analyzing/triaging services, e.g., ECG technicians, medical doctors.

6. Method according to any of claims 2 to 4, **characterized in that**, said analyzing/triaging service (2) is a computer automatically analyzing and classifying said data based on said criteria, wherein said computer automatically uploads said classified data to said host server, said classified data being accessible to said healthcare center/professional (3) through said secured website.

7. Method according to any of the preceding claims, **characterized in that**, a criterion for a low risk level category corresponds to an ECG diagram in time and amplitude space with a normal configuration based on said patient's age and other patient specifics, a criterion for the non-acute risk level category corresponds to an ECG diagram in time and amplitude space slightly deviating from the normal configuration based on said patient's age and other patient specifics, and a criterion for an acute situation corresponds to an ECG diagram in time and amplitude space clearly deviating from the normal configuration based on said patient's age and other patient specifics.

8. System for analyzing patient data transmitted from a patient device (1) to a healthcare center/professional (3) via a secured host server and communication network, **characterized by**, an analyzing apparatus (2) arranged to retrieve said data from said host server, arranged to triage said data into risk level categories based on certain criteria, and arranged to upload said triaged data into said host server.

9. System according to claim 8, **wherein** said patient device is an implant.

10. System according to claims 8 or 9, **wherein** said criteria are defined by said healthcare center/professional (3).

11. System according to any of claims 8 to 10, **wherein**, said apparatus automatically triages said data based on said criteria, wherein if said apparatus recognizes data containing an ECG diagram in time and amplitude space with a normal configuration based on said patient's age and other patient specifics, said apparatus classifies data as 'low risk'; if said apparatus recognizes an ECG diagram in time and amplitude space deviating from the normal configuration based on said patient's age and other patient specifics, said apparatus classifies data as 'non-acute'; and if said apparatus recognizes data containing an ECG diagram in time and amplitude space clearly deviating from the normal configuration based on said patient's age and other patient specifics, said apparatus classifies data as 'acute'.

12. System according to any of claims 8 to 11, **wherein**, said apparatus,
based on said risk level categories of said data (low, non-acute, acute), automatically contacts said healthcare center/professional via email and/or phone, wherein a higher risk level (acute, non-acute) implies a more urgent attention by said apparatus to said healthcare center/professional (3).
